# EUROPEAN PATENT APPLICATION

(11) **EP 4 596 441 A1**
(43) Date of publication of application: **06.08.2025**
(21) Application number: 24305184.4
(22) Date of filing: 02.02.2024
(51) Int. Cl.: B65D 30/24, A61L 2/20, B65D 77/22, B65D 81/20, B65D 77/06

(54) **STORAGE SYSTEMS FOR STERILIZED ITEMS**

(71) Applicant: Becton Dickinson France, 38800 Le Pont-de-Claix (FR)
(72) Inventor: LE LOC'H, Clémentine, 38240 Meylan (FR); EYMERY, Anaïs, 38450 Saint-Georges-de-Commiers (FR)
(74) Representative: Germain Maureau

(57) **Abstract**

Provided herein is a bag including a gas-impervious portion, a porous portion configured to allow a sterilizing gas to penetrate into the bag, a frame surrounding the porous portion, a cap releasably engageable with the frame, and a valve coupled to the cap, wherein the valve is in fluid communication with the porous portion when the cap is engaged to the frame.

## Description

### FIELD OF THE INVENTION

The present disclosure relates generally to storage and transport systems for sterilizable items, and features and methods for detecting a loss of sterilization integrity of such systems.

### BACKGROUND OF THE INVENTION

Various medical devices and related components may be transported from one site to another during manufacturing, assembly, and/or ultimate delivery to a healthcare facility for use. Such devices and components, which may include syringes, medical or surgical instruments, medicine vials or containers, bandaging, or other healthcare items, may be placed inside a bag, sealed, and sterilized prior to transportation or storage - with example sterilization techniques including ethylene oxide (EtO) sterilization and steam sterilization, where a semi-porous bag is used in the sterilization. These sterilization bags are intended to provide a barrier to prevent contaminants, including microorganisms, from entering the bag. The integrity of the sterilization bag has to be maintained until opening for final use of the medical devices or components therein. Otherwise, there exists a risk that contamination of the medical devices occurs, with possibly hazardous consequences for the patient.

A loss of integrity of the sterilization bag may not be easily visible, e.g., in the case of a small breach formed in a wall of the bag. Thus, even if the sterilization bag is seemingly undamaged, the medical devices may have, in fact, been contaminated through even a small breach with limited visibility. In some cases, out of an abundance of caution and in order to counteract such possible contamination of the medical devices, a second sterilization or decontamination process may be carried out at the point of use. However, such potentially-duplicative procedures, which may be applied to all the medical devices contained in a single container or bag, are expensive and time-consuming. In other cases, customer processes at a point of use may not perform an additional sterilization, but instead may require a proof of sterility. The present disclosure provides systems, devices, and methods of use thereof for sterile storage, transport, and visible indication of a compromised environment within one or more containers.

### SUMMARY OF THE INVENTION

The present disclosure provides systems, devices, and methods of use thereof for sterile storage, transport, and visible indication of a compromised environment within one or more containers.

Provided herein is a bag including a gas-impervious portion, a porous portion configured to allow a sterilizing gas to penetrate into the bag, a frame surrounding the porous portion, a cap releasably engageable with the frame, and a valve coupled to the cap, wherein the valve is in fluid communication with the porous portion when the cap is engaged to the frame.

In accordance with some embodiments, the gas-impervious portion is constructed at least in part from low density polyethylene (LDPE), linear low density polyethylene (LLDPE), metallocene polyethylene, biaxially-oriented polyethylene terephthalate (BoPET), or ethylene-vinyl acetate/polyethylene (EVA/PE).

In accordance with some embodiments, the porous portion is constructed at least in part from high-density polyethylene (HDPE) fibers, coated cellulose fibers, or uncoated cellulose fibers.

In accordance with some embodiments, the frame is constructed from polyethylene, acrylonitrile butadiene styrene (ABS), polypropylene, or polycarbonate.

In accordance with some embodiments, the cap is constructed from polyethylene, acrylonitrile butadiene styrene (ABS), polypropylene, or polycarbonate.

In accordance with some embodiments, the valve is a one-way valve configured to permit fluid to exit the porous portion and out of the bag.

In accordance with some embodiments, the cap forms a substantially airtight seal with the frame when the cap is engaged to the frame.

In accordance with some embodiments, the bag further comprises a resealable opening sized to permit one or more medical devices to be placed into the bag.

In accordance with some embodiments, the cap comprises a plurality of locating features formed thereon, positioned about the valve, the locating features functioning to locate a tub sealed within the bag and enable stacking of another bag thereon, in order to stabilize a stack of tubs individually packaged.

Also provided herein is a storage system including a bag comprising a gas-impervious portion, a porous portion configured to allow a sterilizing gas to penetrate into the bag, and a frame surrounding the porous portion. The storage system also includes a container configured to receive the bag therein, the container defining an opening configured to receive at last a portion of the frame therethrough. The storage system further includes a cap releasably engageable with the frame through the opening and a valve coupled to the cap, wherein the valve is in fluid communication with the porous portion when the cap is engaged to the frame.

In accordance with some embodiments, the container is constructed from cardboard or polypropylene.

In accordance with some embodiments, the container includes a substantially planar upper surface and a substantially planar lower surface.

In accordance with some embodiments, the frame forms a friction fit with the opening of the container.

Also provided herein is a process for packaging a medical device. The process includes providing a bag comprising a gas-impervious portion, a porous portion configured to allow a sterilizing gas to penetrate into the bag, and a frame surrounding the porous portion. The process also includes placing the medical device in the bag, sterilizing the medical device by exposing the bag to a sterilizing gas, wherein the sterilizing gas passes through the porous portion. The process further includes engaging a cap to the frame, the cap having a valve coupled thereto, and creating a vacuum within the bag by evacuating gas out of the bag through the valve.

In accordance with some embodiments, the valve is a one-way valve configured to permit gas to exit the porous portion out of the bag, while preventing to enter the bag through the valve.

In accordance with some embodiments, the process further includes placing the bag in a container, the container defining an opening configured to receive at last a portion of the frame therethrough.

In accordance with some embodiments, engaging the cap to the frame includes engaging the cap to the frame through the opening in the container.

In accordance with some embodiments, the process further includes unsnapping the frame from the container and/or unsnapping the cap from the frame, so as to enable re-use of one or more of the container, the cap, or the bag.

In accordance with some embodiments, the bag further comprises a resealable opening, and wherein placing the medical device in the bag includes passing the medical device through the resealable opening.

Also provided herein is a storage system including a bag comprising a gas-impervious portion, a porous portion configured to allow a sterilizing gas to penetrate into the bag, and a frame surrounding the porous portion, the frame comprising a cap integrally formed therewith, with the cap including a valve in fluid communication with the porous portion. The storage system also includes a container configured to receive the bag therein, the container defining an opening configured to receive at last a portion of the frame therethrough, and wherein with the bag positioned within the container, the valve is accessible through the opening.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings, wherein:
FIG. 1 is a partial assembly view of an example of a storage system comprising a bag and single tub, constructed in accordance with the principles of the present disclosure;
FIG. 2 is another partial assembly view of the storage system of FIG. 1, showing the bag in an emptied state and post-sterilization;
FIG. 3A is an assembly view of an example of a storage system comprising a container, bag, and multiple tubs constructed in accordance with the principles of the present disclosure;
FIG. 3B is an isolated view of the bag in the storage system of FIG. 3A, showing a gas permeable, porous portion exploded out therefrom;
FIG. 3C is a side, cross-sectional view of a portion of the storage system of FIG. 3A, showing and
FIG. 4 is a perspective view of an arrangement of a plurality of the storage systems of FIG. 3, placed on a pallet.

### DETAILED DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting as the embodiments or aspects can assume various alternative orientations.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

All documents referred to herein are "incorporated by reference" in their entirety.

The term "at least" is synonymous with "greater than or equal to."

As used herein, "at least one of' is synonymous with "one or more of." For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The word "comprising" and "comprises", and the like, does not exclude the presence of elements or steps other than those listed in any claim or the specification as a whole. In the present specification, "comprises" means "includes" and "comprising" means "including."

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular," or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

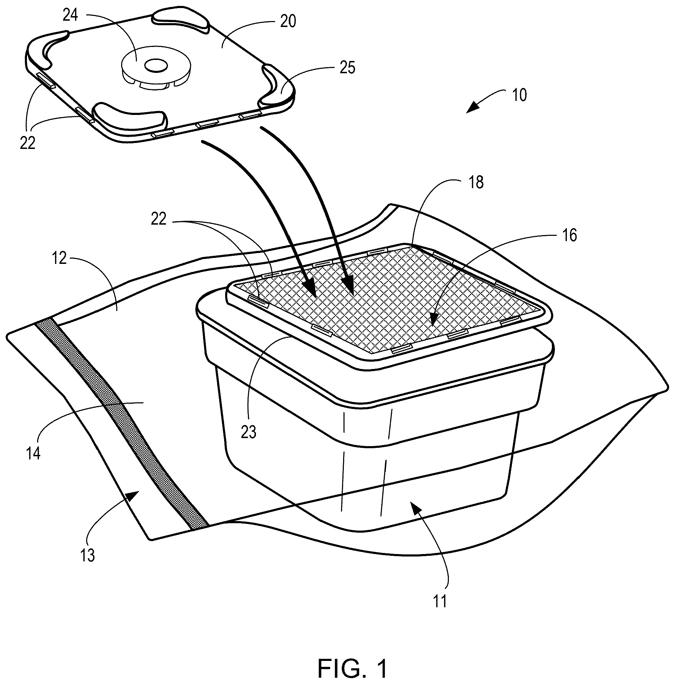

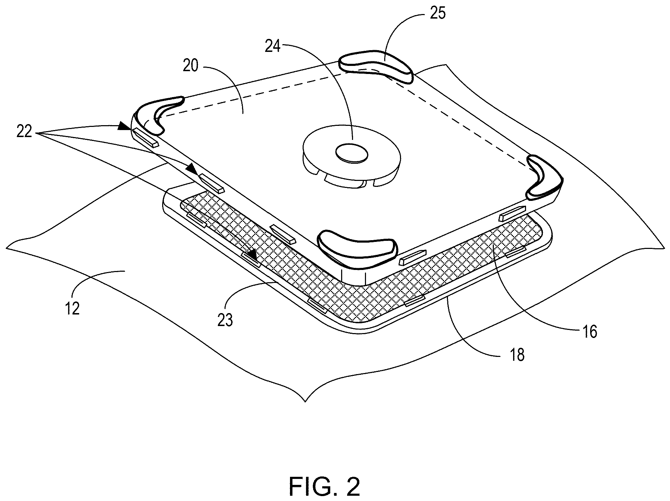

The present disclosure provides systems, devices, and methods of use thereof for sterile storage, transport, and visible indication of a compromised environment within one or more containers. Referring now to the figures in which like reference designations refer to like elements, examples of a system 10 are shown in FIGS. 1-4.

The system 10 may generally include a bag 12 configured or adapted to receive and contain one or more medical devices or components therein, collectively labeled '11' in the accompanying figures. Such devices or components 11 may include, for example, syringes, medical or surgical instruments, medicine vials or containers, bandaging, or other healthcare items. The bag 12 may be sized and shaped to define an inner volume with the capacity to contain a desired quantity of devices or components, and any intermediate packaging or containers, such as a tub (also labeled `11') or otherwise, that the devices or components are contained in. The bag 12 may generally include an outer wall or surface constructed from one or more layers of a film or other material providing the characteristics and feature described herein, such that the outer wall encloses or defines an interior cavity to accommodate the one or more medical devices stored therein. The bag 12 may include one or more openings 13 therein to permit insertion or removal of contents into and out of the bag 12. Such openings 13 may have a resealable closure to permit the bag 12 to be re-used, or alternatively, may have one or more openings 13 permanently sealed or fused after contents are deposited within the bag 12.

The bag 12 may define or include a substantially non-porous, gas-impervious portion 14 and a gas permeable, porous portion 16. "Gas-impervious" refers to a portion with a low permeability to gases. The permeability of the gas-impervious portion 14 may depend on the desired shelf life of the packaging, of the considered gas(es) and of the thickness of the porous portion 16. In one non-limiting example, a low permeability to oxygen is a permeability less than 1,000cm 3< /25µm/24h. The non-porous portion 14 may include one or more layers of polymeric materials, including but not limited to polyethylene, in particular low density polyethylene (LDPE) or linear low density polyethylene (LLDPE), metallocene polyethylene, biaxially-oriented polyethylene terephthalate (BoPET), ethylene-vinyl acetate/polyethylene (EVA/PE), polyethylene/polyamide mixtures (PE/PA/PE), and mixtures thereof.

The porous portion 16 may be adapted or configured to facilitate gas sterilization of the contents of the bag 12, while remaining impermeable to contaminants including microorganisms, bacteria, etc. Such gas sterilization may include, for example, transmission or exposure to ethylene oxide (EtO) through the porous portion 16. By porous, it is meant that the material, thickness, and permeability to gas of the portion 16 are configured to allow a sufficient flow of sterilizing gas to pass through the wall. In one example, the porous portion 16 may be constructed from a nonwoven material made of high-density polyethylene (HDPE) fibers, such as Tyvek, which is frequently used to fulfill the function of the porous wall in a sterilizable bag. In other examples, the nonwoven material may at least partially include coated cellulose fibers and/or uncoated cellulose fibers.

In one example, the non-porous portion 14 has a greater surface area of the bag 12 than the porous portion 16. The surface area of the porous portion 16 may generally be sufficient to allow an efficient decontamination of the contents of the bag 12, while being limited in order to reduce the porosity of the bag 12 after a sterilization process. As one example, for EtO sterilization, the surface area of the porous portion 16 may be configured so as to allow sufficient EtO desorption, As another example, for steam sterilization, the surface area of the porous portion 16 may be configured so as to prevent excess water accumulation.

The bag 12 may include or define a frame 18 at least partially surrounding the porous portion 16. The frame 18 may be constructed from any of a number of suitable materials, including, as non-limiting examples: polyethylene (when the frame 18 is welded to the bag 12), acrylonitrile butadiene styrene (ABS) or polypropylene (when the frame is snapped together around porous portion 16), polycarbonate (to resist steam sterilization) or other similar materials providing sufficient rigidity and structural support to enable the features and characteristics described herein. The frame 18 may be at least partially exposed, mounted, or otherwise extending on an outer surface of the bag 12. The coupling of the frame 18 to the bag 12 may be achieved through an adhesive, thermal molding or welding processes, and/or through the implementation of mechanical attachment mechanisms, including but not limited to clamps, compression elements, snap fit components, or the like.

The system 10 may include a cap 20 engageable with the frame 18 of the bag 12. In one example, the cap 20 may be releasably engageable with the frame 18, and either and/or both of the frame 18 and cap 20 may include one or more attachment features 22 that matably couple to corresponding features on the other component. The attachment features 22 may include, for example, one or more protrusions, openings, channels, grooves, slots, tabs, or otherwise to securely couple the cap 20 to the frame 18. The attachment features 22 may provide for both engagement (i.e., snapping) of the cap 20 with the frame 18 and disengagement (i.e., unsnapping) of the cap 20 from the frame 18, so as to enable re-use of the bag 12 and/or the cap 20.

The cap 20 may be sized and shaped to substantially or entirely cover or seal the porous portion 16 of the bag 12 from the surrounding atmosphere. The cap 20 and/or the frame 18 may include one or more sealing elements 23 to effectuate a substantially air-tight seal between the edges or perimeters of the cap 20 and the frame 18. Such sealing element(s) 23 may include, for example, one or more gaskets, seals, compressible components, coatings, or the like, with the sealing element(s) 23 formed of rubber, thermoplastic elastomers, thermoplastic polyurethane, or silicone, as non-limiting examples. As one example, a sealing element 23 may be provided as an O-ring positioned about frame 18 that forms a seal with cap 20. In another example, the size and dimensions of the frame 18 and cap 20 may be configured to provide a sufficient friction or compression fit as to restrict the flow of air or fluids through the edges or perimeters of the cap 20 and the frame 18, so as to prevent contamination and ensure sterility.

Similar to frame 18, the cap 20 may be constructed from any of a number of suitable materials, including, as non-limiting examples: polyethylene, acrylonitrile butadiene styrene (ABS) or polypropylene, or polycarbonate, as non-limiting examples . The cap 20 may provide sufficient rigidity and/or strength to resist impact or other potentially damaging forces experienced by the cap 20, bag 12, and/or other components of the system 10 during transport or storage.

The cap 20 may include a valve 24 attached on or about a portion of the cap 20. The valve 24 may be adapted or configured to facilitate and/or regulate a portion of the fluid flow coming from the inside bag 12 and through porous portion 16. The valve 24 may include or define a one-way valve permitting fluid flow in a single direction, while preventing fluid flow in an opposite direction. Examples of one-way valve mechanisms may include check valves, flapper valves, ball-and-spring configurations, rotary valves, or the like. The valve could also comprise a silicone membrane used as a flap. In some embodiments, the valve may include a cap thereon (e.g., polypropylene cap) that seals the valve after vacuum. The direction of fluid flow through the valve 24 may be configured to exhaust or remove fluidfrom an interior of the bag 12 to create a vacuum therein. In one example, when the cap 20 is coupled to the frame 18, the valve 24 may permit fluid flow from an interior of the bag 12, through the porous portion 16, and out of the valve 24 into the surrounding atmosphere. The valve 24 may also restrict or prevent fluid from ingressing into and towards the porous portion 16 and into the interior of the bag 12. The valve 24 may be sized, configured, or otherwise adapted to provide sufficient fluid flow rates therethrough to enable efficient evacuation of fluid from the bag 12, as described herein. Accordingly, the valve 24 is able to provide evidence of tampering after creation of a vacuum within bag 12.

In some embodiments, the cap 20 may also include locating features 25 formed thereon that can enable stacking of multiple bags 12 and tubs 11. The locating features 25 may be formed as a plurality of bumps or protrusions that extend upwardly from a top surface of the cap 20, with the locating features 25 being positioned about the valve 24, such as at the four corners of the cap 20. The locating features 25 may function to assist with locating a tub 11 sealed within the bag 12 and enable stacking of another bag 12 thereon, in order to stabilize a stack of tubs 11 individually packaged. In the illustrated embodiment, the locating features 25 are formed on cap 20 such that, when another tub 11 (in a bag 12) is stacked onto the illustrated tub 11 (in bag 12), the bottom of the tub 11 will fit onto and engage the arrangement of locating features 25 on cap - thereby aligning and stabilizing the stacked tub 11 (in a bag 12) on the original tub 11 and bag 12.

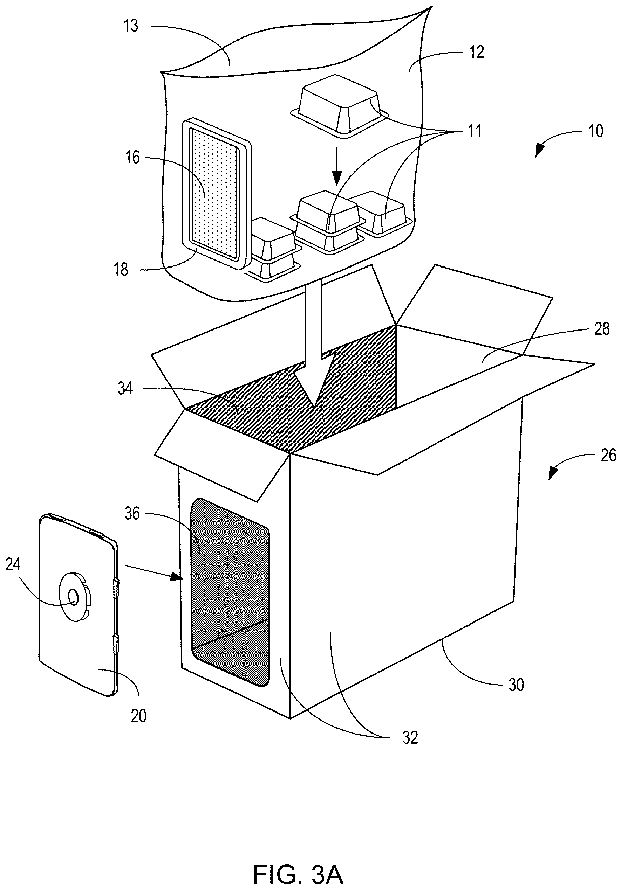

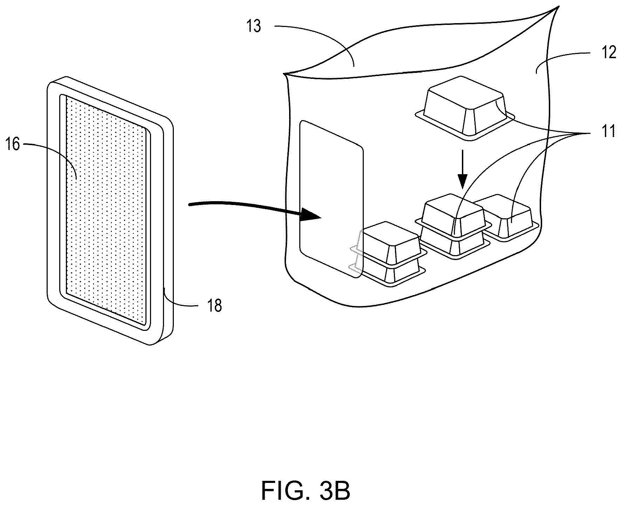

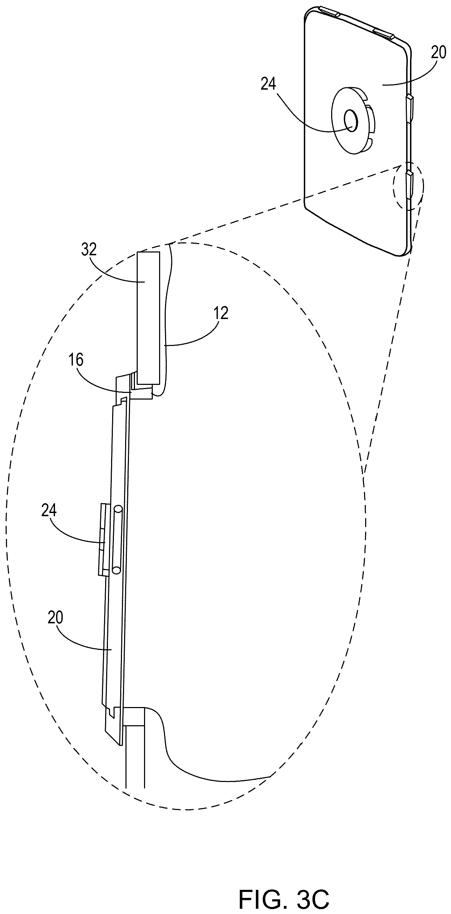

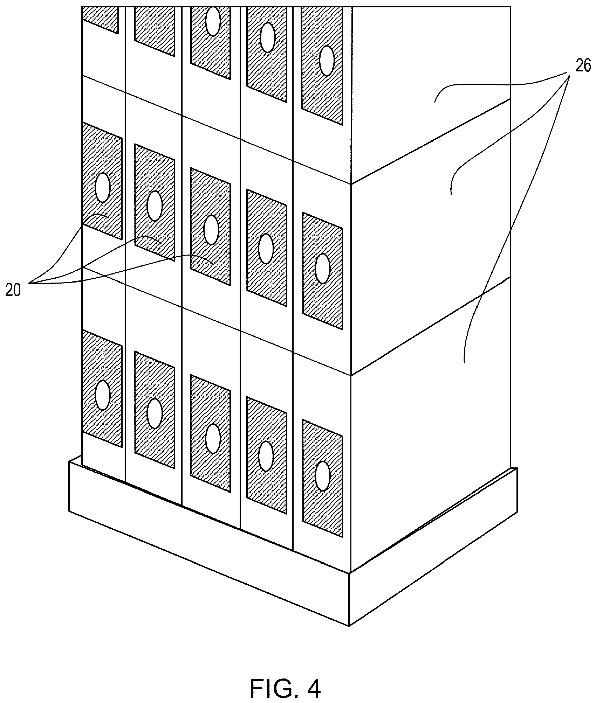

Now referring to FIGS. 3A, 3B, 3C and 4, the system 10 may include a container 26 adapted or configured to receive the bag 12 at least partially therein. The container 26 may include an upper portion 28, a lower portion 30, one or more sidewalls 32 extending between the upper and lower portions, and a cavity 34 defined therein. The upper portion 28 and/or lower portion 30 may define or include one or more substantially planar surfaces to enable a plurality of similarly configured containers to be stacked upon one another. The container 26 may include or define an opening 36 in one or more of the sidewalls 32, with the opening 36 adapted or configured to receive at least a portion of the frame 18 and/or porous portion 16 therethrough. The opening 36 may be configured or adapted to enable the cap 20 to attach to or otherwise engage the frame 18 and/or porous portion 16 through the opening 36, such as by the frame 18 snapping onto the walls 32. The container 10 may include one or more protrusions, openings, channels, grooves, slots, tabs, or otherwise to attach to similar and/or complimentary features on the bag 12, frame 18, and/or the cap 20 to secure the position or relative arrangement of the components of the system 10 disclosed herein. In some embodiments, in addition to the frame 18 being snapped onto the container 26, the frame 18 may also be unsnapped from the container 26, so as to enable re-use of the container 26 and/or or the bag 12.

The container 26 may be constructed from one or more of cardboard and/or akylux (polypropylene). The container 26 may provide sufficient rigidity and/or strength to resist impact, sterilization process conditions (e.g., heat and humidity resistance), or other potentially damaging forces experienced by the cap 20, bag 12, and/or other components of the system 10 during transport or storage, and may have sufficient rigidity or strength to support multiple containers in a stacked configuration, as shown in FIG. 4.

In an example of use of the system 10, one or more devices or components may be inserted or positioned into the bag 12. The bag 12 may be sealed, for example, by closing a resealable opening, or by fusing or permanently sealing an opening otherwise defined by the bag 12. The contents of the bag 12 may then be sterilized. Such sterilization may be achieved, for example, by exposing the porous portion 16 of the bag to a sterilizing gas such that the sterilizing gas passes through the porous portion 16 and sterilizes the contents of the bag 12.

The cap 20 may be coupled to (e.g,. snapped onto) the frame 18 either in a stand-alone configuration as shown in FIGS. 1-2, or the bag 12 may be placed in the container 26, with the cap 20 engaging the frame 18 through the opening 36. Once the cap 20 is secured to the frame 18 of the bag 12, a vacuum pump or other fluid extraction device (not shown) may be attached to the valve 24 and actuated to evacuate air, sterilization gas, or other fluid from the bag 12, out of the porous portion 16, and through the valve 24 of the cap 20, so as to create a vacuum for tamper evidence. The bag 12 may, for example, be evacuated until the bag 12 is substantially collapsed around the interior contents of the bag 12, indicating that all or most gas/fluid has been removed from the bag 12. Once excess gas or fluid has been sufficiently evacuated from the bag 12, the bag 12 may be transported and/or stored for future use.

In other embodiments, rather than cap 20 being coupled to the frame 18, the cap 20 may instead be integrally formed with the frame 18. In such a configuration, with the bag 12 positioned within the container 26, the valve 24 may be accessible through the opening 36 - such that sterilization and vacuum creation may be performed without having to unpack the bag 12 from container 26.

Upon reaching a destination or otherwise being ready for use, the bag 12 can be visually inspected to ascertain whether the evacuated condition is still present, which is a direct indication of whether the interior sterilized environment of the bag 12 has been compromised. For example, even a small hole or breach of the bag 12 will remove the vacuum state therein, causing the bag 12 to 'relax' or otherwise expand from the evacuated, collapsed state, and will be readily visible without requiring close-up inspection of the entire surface of the bag 12.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A bag, comprising:
a gas-impervious portion;
a porous portion configured to allow a sterilizing gas to penetrate into the bag;
a frame surrounding the porous portion;
a cap releasably engageable with the frame; and
a valve coupled to the cap, wherein the valve is in fluid communication with the porous portion when the cap is engaged to the frame.

2. The bag of claim 1, wherein the gas-impervious portion is constructed at least in part from low density polyethylene (LDPE), linear low density polyethylene (LLDPE), metallocene polyethylene, biaxially-oriented polyethylene terephthalate (BoPET), or ethylene-vinyl acetate/polyethylene (EVA/PE).

3. The bag of claim 1, wherein the porous portion is constructed at least in part from high-density polyethylene (HDPE) fibers, coated cellulose fibers, or uncoated cellulose fibers.

4. The bag of claim 1, wherein the frame is constructed from polyethylene, acrylonitrile butadiene styrene (ABS), polypropylene, or polycarbonate.

5. The bag of claim 1, wherein the cap is constructed from polyethylene, acrylonitrile butadiene styrene (ABS), polypropylene, or polycarbonate.

6. The bag of claim 1, wherein the valve is a one-way valve configured to permit fluid to exit the porous portion and out of the bag.

7. The bag of claim 1, wherein the cap forms a substantially airtight seal with the frame when the cap is engaged to the frame.

8. The bag of claim 1, wherein the bag further comprises a resealable opening sized to permit one or more medical devices to be placed into the bag.

9. The bag of claim 1, wherein the cap comprises a plurality of locating features formed thereon, positioned about the valve, the locating features functioning to locate a tub sealed within the bag and enable stacking of another bag thereon, in order to stabilize a stack of tubs individually packaged.

10. A storage system, comprising:
a bag, the bag comprising:
a gas-impervious portion;
a porous portion configured to allow a sterilizing gas to penetrate into the bag; and
a frame surrounding the porous portion;
a container configured to receive the bag therein, the container defining an opening configured to receive at last a portion of the frame therethrough;
a cap releasably engageable with the frame through the opening; and
a valve coupled to the cap, wherein the valve is in fluid communication with the porous portion when the cap is engaged to the frame.

11. The storage system of claim 9, wherein the container is constructed from cardboard or polypropylene.

12. The storage system of claim 9, wherein the container includes a substantially planar upper surface and a substantially planar lower surface.

13. The storage system of claim 9, wherein the frame forms a friction fit with the opening of the container.

14. A process for packaging a medical device comprising:
providing a bag, the bag comprising:
a gas-impervious portion;
a porous portion configured to allow a sterilizing gas to penetrate into the bag; and
a frame surrounding the porous portion;
placing the medical device in the bag;
sterilizing the medical device by exposing the bag to a sterilizing gas, wherein the sterilizing gas passes through the porous portion;
engaging a cap to the frame, the cap having a valve coupled thereto;
creating a vacuum within the bag by evacuating gas out of the bag through the valve.

15. The process of claim 13, wherein the valve is a one-way valve configured to permit gas to exit the porous portion out of the bag, while preventing to enter the bag through the valve.

16. The process of claim 13, further comprising placing the bag in a container, the container defining an opening configured to receive at last a portion of the frame therethrough.

17. The process of claim 15, wherein engaging the cap to the frame includes engaging the cap to the frame through the opening in the container.

18. The process of claim 13, further comprising unsnapping the frame from the container and/or unsnapping the cap from the frame, so as to enable re-use of one or more of the container, the cap, or the bag.

19. The process of claim 13, wherein the bag further comprises a resealable opening, and wherein placing the medical device in the bag includes passing the medical device through the resealable opening.

20. A storage system, comprising:
a bag, the bag comprising:
a gas-impervious portion;
a porous portion configured to allow a sterilizing gas to penetrate into the bag; and
a frame surrounding the porous portion, the frame comprising a cap integrally formed therewith, with the cap including a valve in fluid communication with the porous portion; and
a container configured to receive the bag therein, the container defining an opening configured to receive at last a portion of the frame therethrough; and
wherein, with the bag positioned within the container, the valve is accessible through the opening.
